(19) Europäisches Patentamt

European Patent Office

Office européen des brevets

(11) **EP 1 492 524 B1**

(12) **EUROPÄISCHE PATENTSCHRIFT**

(45) Veröffentlichungstag und Bekanntmachung des Hinweises auf die Patenterteilung:
**26.04.2006 Patentblatt 2006/17**

(51) Int Cl.:
*A61K 31/375* (2006.01)          *A61K 31/355* (2006.01)
*A61K 31/07* (2006.01)          *A61P 35/00* (2006.01)

(21) Anmeldenummer: **03745769.4**

(22) Anmeldetag: **27.03.2003**

(86) Internationale Anmeldenummer:
**PCT/EP2003/003205**

(87) Internationale Veröffentlichungsnummer:
**WO 2003/084531 (16.10.2003 Gazette 2003/42)**

(54) **VITAMIN-KOMBINATION ZUM SCHUTZ BEI CHEMO- ODER/UND STRAHLUNGSTHERAPIE MALIGNER TUMOREN**

VITAMIN COMBINATION FOR PROVIDING PROTECTION DURING THE CHEMOTHERAPY AND/OR RADIOTHERAPY OF MALIGNANT TUMOURS

COMBINAISON DE VITAMINES POSSEDANT UN EFFET PROTECTEUR PENDANT LA CHIMIOTHERAPIE ET/OU LA RADIOTHERAPIE DE TUMEURS MALIGNES

(84) Benannte Vertragsstaaten:
**AT BE BG CH CY CZ DE DK EE ES FI FR GB GR HU IE IT LI LU MC NL PT RO SE SI SK TR**

(30) Priorität: **10.04.2002 DE 10215765**

(43) Veröffentlichungstag der Anmeldung:
**05.01.2005 Patentblatt 2005/01**

(73) Patentinhaber: **APOCARE Pharma GmbH**
**33647 Bielefeld (DE)**

(72) Erfinder:
• **MOROZKINA, Tatjana Sergejevna**
**Minsk, 220056 (BY)**
• **Sukolinski, Vladimir Nikolajevich**
**Dorf "Borovljany",**
**Minsker Rayon, 223053 (BY)**

(74) Vertreter: **Huber, Bernhard et al**
**Weickmann & Weickmann**
**Patentanwälte**
**Postfach 86 08 20**
**81635 München (DE)**

(56) Entgegenhaltungen:
• **K.N.PRASAD E.A.: "High doses of multiple antioxidant vitamins: essential ingredients in improving the efficacy of standard cancer therapy" JOURNAL OF THE AMERICAN COLLEGE OF NUTRITION, Bd. 18, Nr. 1, 1999, Seiten 13-25, XP002223248**
• **W.OOSTERLINCK: "The management of superficial bladder cancer" BJU INTERNATIONAL, Bd. 87, Nr. 2, 2001, Seiten 135-140, XP001159180**
• **D.K.BENOVA: "Anticlastogenic effects of a polyvitamin product, "Pharmavit", on gamma-ray induction of somatic and germ cell chromosome aberrations in the mouse" MUTATION RESEARCH, Bd. 269, Nr. 2, 1992, Seiten 251-258, XP008018894**
• **J.A.DRISCO E.A.: "The use of antioxidant therapies during chemotherapy" GYNECOLOGIC ONCOLOGY, Bd. 88, Nr. 3, 2003, Seiten 434-439, XP001159181**

## Beschreibung

Die vorliegende Erfindung bezieht sich auf die Verwendung eines Mittels zur Verbesserung der Verträglichkeit von Chemotherapie oder/und Strahlungstherapie maligner Tumoren.

Das Auftreten maligner Tumoren hat in den letzten 20 Jahren unaufhaltsam zugenommen. Studien an Patienten mit Magenkrebs in unterschiedlichen Stadien haben gezeigt, dass bei Patienten mit Magenkrebs im Frühstadium eine chirurgische Entfernung des malignen Tumors zweckmäßig ist, dass bei weiter fortgeschrittenem Krebsstadium die alleinige chirurgische Behandlung jedoch nicht effektiv ist. Bei 20 bis 30 % solcher Patienten treten im ersten Jahr nach der Operation Lokalrezidiven und Fernmetastasen auf, innerhalb des zweiten Beobachtungsjahres beträgt der prozentuale Anteil bereits 40 %. Die durchschnittliche 5-jährige Überlebensrate von Patienten mit Dickdarmkrebs, die chirurgisch behandelt worden waren, beträgt etwa 50 %. Die meisten der beobachteten Patienten weisen Lokalrezidive und Fernmetastasen auf. Es hat sich gezeigt, dass neben der chirugischen Behandlung Antitumortherapien wie die Chemotherapie bzw. Polychemotherapie oder/und Strahlungstherapie erforderlich sind.

Bei der Strahlungstherapie wird ionisierende Strahlung mit dem Ziel der Schädigung des Tumorgewebes angewendet. Ionisierende Strahlung (z.B. Gamma- oder Röntgenstrahlung) kann allerdings durch Erzeugung von Radikalen auch eine Zerstörung von gesundem Gewebe verursachen. Bei wirksamen Strahlungsdosen treten meist starke Strahlungskomplikationen auf, sodass die Bestrahlungstherapie normalerweise mit deutlich geringeren Dosen durchgeführt wird als es ein optimaler Effekt der Therapie erfordern würde. Als Strahlungskomplikationen treten beispielsweise Strahlendermatitis, Lungenfibrose, Schleimhautschäden im Magen-DarmTrakt, verstärkte Schmerzen und Immunodepressionen auf. Da die Bestrahlung auch gesundes Gewebe angreift, wird der Therapie-Erfolg der Bestrahlung beträchtlich gemindert, was zu einer geringen Lebenserwartung der betroffenen Patienten führt. Studien haben ergeben, dass beispielsweise bei Patienten mit Tumoren in fortgeschrittenem Stadium Dosen von 60 Gy erforderlich sind, um einen guten Effekt zu erzielen. Solche hohen Dosen sind allerdings aufgrund der beträchtlichen Nebenwirkungen ohne Schutz des Organismus nicht vertretbar. Angaben über Ergebnisse von Strahlungstherapien sind bei Jarmonenco (Radiobiologie: Menschen und Tiere) zu finden. Die folgende Tabelle I, die dieser Literaturstelle entnommen ist, zeigt die Toleranzdosis bei ionisierender Bestrahlung für lebenswichtige Organe.

Tabelle I

| Organ | Maximal. EFD (Gy) | FZD | Max. Isoäquivalenz (Gy) | Toleranzdosis (Gy) |
|---|---|---|---|---|
| Haut | 7,2 | 119 | 72 | 50-70 |
| Rückenmark | 3,3 | 35,5 | 21 | 50-57 |
| linker Leberlappen | 7,1 | 115,6 | 70 | 30-50 |
| linke Niere | 6,7 | 107 | 64 | 13-25 |
| Bauchspeicheldrüse: | | | | |
| -Kopf | 6,9 | 111 | 67 | - |
| -Körper | 5,4 | 76 | 46 | 30-100 |
| -Schwanz | 2,5 | 23 | 14 | - |
| Dünndarm | 5,4 | 76 | 46 | 35-45 |

Maximal. Einzelfokusdosis (EFD):  Absorbierte Dosis für die in der Tabelle angegebenen Organe nach jeder Strahlungsbehandlung (5 Behandlungen) mit einer EFD von 7 Gy

Faktor-Zeit-Dosis (FZD):  Zur Umrechnung des Schadeneffekts im Gewebe nach der Bestrahlung mit höheren Fraktionen (7 Gy) im Vegleich zu den Standardmethoden (EFD = 2 Gy).

Max. Isoäquivalenz:  Gesamte absorbierte Dosis für verschiedene Organe nach 5 Strahlungsbehandlungen (berechnet nach speziellen radiologischen Tabellen FZD).

Toleranz-Dosis:  Maximal verträgliche Bestrahlungsdosierung für verschiedene Organe ohne Strahlenschutzmittel.

Ein durch die Bestrahlung stimulierbarer Oxidationsprozess ist z.B. die Lipidperoxidation, bei der freie Radikale zu einer Zersetzung von Lipiden führen. Dabei bilden sich Lipidperoxidations-Produkte, wie z.B. Malondialdehyd (MDA) und 4-Hydroxy-2-trans-hexenal. Lipidperoxidations-Produkte sind meist mutagene Substanzen, die den Organismus schädigen können. Die durch Bestrahlung induzierte Oxidation im Körper kann zur Zerstörung von gesundem Gewebe, DNA und Zellmembranen führen.

Bisher wurden zum Schutz von gesundem Gewebe bei der Strahlungstherapie Mittel wie z.B. Mexamin und Cysteamin-Hydrochlorid eingesetzt. Aufgrund der Radikalfängereigenschaften von Cysteamin (2-Aminoethanthiol) hat es in der Behandlung von Strahlenschäden Verwendung gefunden. Diese herkömmlichen Strahlenschutzmittel weisen allerdings eine hohe Toxizität auf, was zu beträchtlichen Nebenwirkungen führt. Die Dosen an Strahlenschutzmittel, die verabreicht werden müssten, um eine effektive Strahlungstherapie durchführen zu können, wären gesundheitlich nicht zu vertreten. Dadurch wird die Anwendungsbreite und -dauer enorm eingeschränkt.

Bei der Chemotherapie bzw. Polychemotherapie werden Zellgifte verabreicht, die spezifisch in das Wachstum des Tumorgewebes eingreifen sollen. Herkömmlicherweise werden als Cytostatika z.B. 5-Fluoruracil (5-Fu), cis-Platin und Doxorubicin eingesetzt. Diese Mittel weisen aber eine geringe Selektivität für das Tumorgewebe auf und schädigen daher gesundes Gewebe in hohem Ausmaß. Durch die im Allgemeinen verwendeten Cytostatika kann durch das Auftreten von freien Radikalen im Organismus die Lipidperoxidation initiiert werden. Die auftretenden Nebenwirkungen der Chemotherapie schränken also die einsetzbare Dosis dieser Mittel und somit die Effektivität der Antitumortherapie ein.

Wie es bei den Einzeltherapien der Fall ist, treten auch bei der kombinierten Strahlungs/Chemotherapie Schädigungen von gesunden Partien des Organismus auf. Auch bei alleiniger chirurgischer Behandlung von Tumorpatienten wird der ohnehin bereits geschwächte Organismus der Kranken zusätzlichem Stress ausgesetzt, wodurch z.B. Oxidationsprozesse im Körper stimuliert werden können.

Ein großes Problem bei der Behandlung maligner Tumoren durch chirurgische Eingriffe oder/und Chemo- oder/und Strahlungstherapie besteht also darin, dass diese Therapien eine zusätzliche Belastung für die Kranken darstellen. Es werden auch gesunde Partien des Organismus geschädigt, da die üblicherweise verwendeten Cytostatika unselektiv sind und die Bestrahlungen auf den gesamten Organismus einwirken können.

Die Aufgabe der vorliegenden Erfindung war, die Verträglichkeit von chirurgischen Eingriffen bei malignen Tumoren und der Chemotherapie oder/und Strahlungstherapie maligner Tumoren zu verbessern.

Gelöst wird diese Aufgabe überraschenderweise durch die Verwendung einer bestimmten Kombination aus Ascorbinsäure, Tocopherol und Retinol, wobei Ascorbinsäure frei und Tocopherol und Retinol frei oder/und in Form ihrer pharmazeutisch annehmbaren Ester vorliegen und in einem Masseverhältniszueinandervon 1,5-2,5:0,3-0,8: 0,028-0,033, bezogen auf Ascorbinsäure, α-Tocopherol als Acetat und Retinol als Acetat, enthalten sind, zur Herstellung einer Arzneimittels zur Verbesserung der Verträglichkeit von chemotherapie oder/und Strahlungstherapie maligner Tumoren.

Durch die vorliegende Erfindung wird des Weiteren ein Verfahren zur Herstellung eines Mittels bereitgestellt, das die Verträglichkeit von Chemotherapie oder/und Strahlungstherapie maligner Tumoren verbessert und dadurch gekennzeichnet ist, dass man eine Kombination aus Ascorbinsäure, Tocopherol und Retinol, wobei Ascorbinsäure frei, Tocopherol und Retinol frei oder/und in Form ihrer pharmazeutisch annehmbaren Ester, in einem Masseverhältnis zueinander von 1,5-2,5:0,3-0,8:0,028-0,033, bezogen auf Ascorbinsäure, α-Tocopherol als Acetat und Retinol als Acetat, vorliegen, als Wirkstoff zubereitet.

Ferner umfasst die vorliegende Erfindung ein Verfahren zur Verbesserung der Verträglichkeit von Chemotherapie oder/und Strahlungstherapie maligner Tumoren, das dadurch gekennzeichnet ist, dass ein Mittel verabreicht wird, das eine Kombination aus Ascorbinsäure, Tocopherol und Retinol enthält, wobei Ascorbinsäure frei, Tocopherol und Retinol frei oder/und in Form ihrer pharmazeutisch annehmbaren Ester vorliegen und in einem Masseverhältnis zueinander von 1,5-2,5:0,3-0,8:0,028-0,033, bezogen auf Ascorbinsäure, α-Tocopherol als Acetat und Retinol als Acetat, enthalten sind.

Des Weiteren betrifft die Erfindung eine Kombination aus Ascorbinsäure, Tocopherol und Retinol, wobei Ascorbinsäure frei und Tocopherol und Retinol frei oder/und in Form ihrer pharmazeutisch annehmbaren Ester vorliegen und in einem Masseverhältnis zueinander von 1,5-2,5:0,3-0,8:0,028-0,033, bezogen auf Ascorbinsäure, α-Tocopherol als Acetat und Retinol als Acetat, enthalten sind, zur Verwendung zur Herstellung eines Arzneimittels zur Verbesserung der Verträglichkeit von Chemotherapie oder/und Strahlungstherapie maligner Tumoren.

Das Mittel enthält Ascorbinsäure, Tocopherol und Retinol in einem Masseverhältnis zueinander von 1,5-2,5 : 0,3-0,8 : 0,028 - 0,033 vorzugsweise von 1,6-2,3:0,4-0,68:0,029-0,032, mehr bevorzugt von 1,8-2,1 : 0,5-0,65 : 0,030-0,032, besonders bevorzugt von 2 : 0,6 : 0,033, bezogen auf Ascorbinsäure, α-Tocopherol als Acetat und Retinol als Acetat.

Die Mindestdosis von Ascorbinsäure, Tocopherol und Retinol beträgt im Allgemeinen 150 mg/Tag, 50 mg/Tag bzw. 3 mg/Tag, bezogen auf Ascorbinsäure, α-Tocopherol als Acetat und Retinol als Acetat. Bevorzugt werden mindestens 1,5 g/Tag, 300 mg/Tag bzw. 28 mg/Tag verabreicht. Bei Wahrung des erfindungsgemäßen Verhältnisses der Vitamine zueinander kann die Dosis je nach Bedarf nach oben hin eingestellt werden, die Dosisobergrenzen liegen aber im Allgemeinen bei 2,5 g/Tag, 800 mg/Tag bzw. 33 mg/Tag, bezogen auf Ascorbinsäure, α-Tocopherol als Acetat und Retinol als Acetat.

Gemäß vorliegender Erfindung ist es des Weiteren möglich, der erfindungsgemäßen Vitamin-Kombination β-Carotin zuzusetzen, wodurch die Menge an eingesetztem Retinol verringert werden kann. Dies kann von Vorteil sein, um die Langzeitverträglichkeit der erfindungsgemäßen Vitamin-Kombination beim Patienten zu erhöhen.

Ein weiterer Aspekt der vorliegenden Erfindung ist daher die Verwendung einer Kombination aus Ascorbinsäure, Tocopherol, Retinol und β-Carotin, wobei Ascorbinsäure frei und Tocopherol und Retinol frei oder/und in Form ihrer pharmazeutisch annehmbaren Ester und β-Carotin frei vorliegen und in einem Masseverhältnis zueinander von 1,5-2,5: 0,3-0,8:0,028-0,033, bezogen auf Ascorbinsäure, α-Tocopherol als Acetat und Retinol als Acetat zusammen mit β-Carotin, enthalten sind, als Mittel zur Verbesserung der Verträglichkeit von Chemotherapie oder/und Strahlungstherapie maligner Tumoren.

Noch ein weiterer Aspekt der vorliegenden Erfindung ist eine Kombination aus Ascorbinsäure, Tocopherol, Retinol und β-Carotin, wobei Ascorbinsäure frei und Tocopherol und Retinol frei oder/und in Form ihrer pharmazeutisch annehmbaren Ester und β-Carotin frei vorliegen und in einem Masseverhältnis zueinander von 1,5-2,5:0,3-0,8: 0,028-0,033, bezogen auf Ascorbinsäure, α-Tocopherol als Acetat und Retinol als Acetat zusammen mit β-Carotin enthalten sind, zur Verwendung als Mittel zur Verbesserung der Verträglichkeit von Chemotherapie oder/und Strahlentherapie maligner Tumoren.

Erfindungsgemäß ist die Gesamtmenge an Retinol und β-Carotin vorzugsweise aus 1/3 Retinol und 2/3 β-Carotin zusammengesetzt, wobei der erfindungsgemäße Masseanteil an Retinol in der Vitamin-Kombination in diesen Aspekt der Erfindung durch die Summe der Retinol- und β-Carotin-Anteile eingehalten wird. Vorzugsweise beträgt die Tagesdosis an mit der erfindungsgemäßen Vitamin-Kombination verabreichtem β-Carotin 10 bis 25 mg, mehr bevorzugt 15 bis 20 mg, besonders bevorzugt 16 bis 19 mg. Die verabreichten Dosen an Ascorbinsäure, Tocopherol und Retinol liegen bevorzugt in den oben definierten Bereichen.

Ascorbinsäure (Vitamin C) ist ein wasserlösliches Vitamin, das vor allem in frischen Früchten und Gemüse, wie z.B. Hagebutten, Orangen, Zitronen und Paprika enthalten ist. Ein Vitamin-C-Mangel (Hypovitaminose C) führt zu Skorbut (Haut- und Zahnfleischbluten bis zu Zahnausfall), Infektionsanfälligkeit sowie Wachstumsstörungen bei Kindern. Ascorbinsäure wird beispielsweise als Antioxidans in Lebensmitteln verwendet, wobei die Antioxidationswirkung vorwiegend darin besteht, dass Ascorbinsäure als Radikalfänger wirken kann.

Tocopherole (Vitamin E) sind fettlösliche Vitamine, die in z.B. Leber und Fettgewebe gespeichert werden können. Von den verschiedenen Tocopherolen, wie z.B. α-, β-, γ- und δ-Tocopherol, wird meist das α-Tocopherol (einschließlich seiner Ester) als das eigentliche Vitamin E bezeichnet. Vitamin E hat Einfluss auf den Arachidonsäure-Stoffwechsel (Hemmung der Thromboxan-Biosynthese) und wirkt entzündungshemmend. Hauptsächlich scheint Vitamin E durch Abfangen von Hyperoxiden und Peroxiden als Antioxidans für ungesättigte Fettsäuren, Vitamin A und Carotine zu fungieren. Bei Vitamin-E-Mangel kann erhöhte Lipidperoxidation auftreten und es können Muskelschwäche und neurodegenerative Veränderungen festgestellt werden.

Retinol (Vitamin A) und die Ester davon sind auch fettlöslich und spielen beim Aufbau des Sehpurpurs (Rhodopsin) in der Netzhaut eine wichtige Rolle. Daher kann der Mangel an diesem Vitamin zu Schädigungen der Binde- und Hornhaut der Augen führen. Durch Vitamin-A-Mangel können außerdem Wachstumsstörungen und eine erhöhte Infektionsanfälligkeit ausgelöst werden. Vitamin A besitzt Antioxidans-Eigenschaften, allerdings kann eine übermäßige Aufnahme von Vitamin A zu Krankheitserscheinungen wie Kopfschmerzen und Übelkeit, aber auch zu Haarausfall, Knochenschwellungen etc. führen.

β-Carotin stellt das eigentliche Provitamin A dar und ist das wichtigste der zur Gruppe der Carotinoiden gehörenden Carotine. Es kommt beispielsweise in Möhren und anderen Gemüsen vor und wird unter anderem als Antioxidans und Farbstoff eingesetzt.

Die erfindungsgemäße Vitamin-Kombination kann ferner für Pharmazeutika übliche Zusatzstoffe wie Bindemittel, Geschmacksstoffe, Farbstoffe oder/und Süßungsmittel umfassen.

In der Vitamin-Kombination kann L-(+)-Ascorbinsäure (Vitamin C) in freier Form, Tocopherol und Retinol können sowohl frei als auch in Form ihrer pharmazeutisch annehmbaren Ester eingesetzt werden. β-Carotin wird gegebenenfalls in freier Form eingesetzt. Bevorzugt werden Tocopherol oder/und Retinol in Form ihrer pharmazeutisch annehmbaren Ester, wie z.B. Essigsäure- und Bernsteinsäureester eingesetzt, besonders bevorzugt werden die entsprechenden Acetate verwendet.

Tocopherol (Vitamin E) kann in der Vitamin-Kombination als α-, β-, γ oder/und δ-Tocopherol enthalten sein, wobei bevorzugt α- oder/und γ-Tocopherol, besonders bevorzugt α-Tocopherol eingesetzt wird.

Durch die Verwendung der erfindungsgemäßen Vitamin C, E, A-Kombination bei Anti-Tumorbehandlungen wie z.B. der Chemo- oder/und Strahlungstherapie kann eine Verbesserung der Verträglichkeit und somit der Effektivität solcher Therapien erreicht werden.

Es hat sich überraschenderweise gezeigt, dass das Verhältnis der Vitamine zueinander entscheidend für die Wirkung der Kombination ist. Es wurde außerdem gefunden, dass die Tagesdosis der jeweiligen Vitamine (und gegebenenfalls Provitamin) vorteilhafterweise in einem bestimmten Bereich liegt (s.o.). Dabei wurde ein sogenannter "No-Effekt-Level" ermittelt, unterhalb dessen im Allgemeinen keine Wirksamkeit mehr beobachtet wird. Die Werte für die Vitamine C, E und A in der erfindungsgemäßen Vitamin-Kombination betragen hierfür etwa 150 mg/Tag, 50 mg/Tag bzw. 3 mg/Tag (s.o.).

Die Vitamine sollten gleichzeitig, teilweise gleichzeitig oder mit nicht allzu großer Zeitverzögerung nacheinander eingenommen werden. Dabei können die Vitamine als Tabletten, Kapseln, Pulver oder in einer sonstigen üblichen Darreichungsform mit der entsprechenden Dosierung zugeführt bzw. eingenommen werden. Eine Variante besteht darin, eine Tablette oder Kapsel zu verwenden, die Ascorbinsäure, Tocopherol und Retinol mit dem entsprechenden Masseverhältnis umfasst, wobei der Kern der Tablette bzw. Kapsel die fettlöslichen Vitamine E und A umfasst und dieser Kern umgeben ist von einer Schicht, die das wasserlösliche Vitamin C umfasst. Das Vitamin-Mittel kann oral oder/und parenteral eingenommen bzw. verabreicht werden. Weiterhin ist die Verabreichung mittels Injektion, insbesondere intramuskulär, subkutan oder/und intravenös erfindungsgemäß vorgesehen. In einer bevorzugten Ausführungsform wird die erfindungsgemäße Vitamin-Kombination intramuskulär verabreicht, was insbesondere bei Patienten, die (zeitweise) nur parenteral ernährt werden können, gegenüber der oralen Verabreichung von Vorteil sein kann. Hierbei ist weiterhin bevorzugt, die Vitamine E und A (gegebenenfalls plus Provitamin A) intramuskulär zu verabreichen, das wasserlösliche Vitamin C jedoch bevorzugt subkutan oder intravenös zuzuführen.

Zum Schutz von gesundem Gewebe und zur Effektivitätssteigerung der entsprechenden Antitumortherapie kann erfindungsgemäß die Vitamin-Kombination vor, während oder/und nach einer solchen Therapie verabreicht werden.

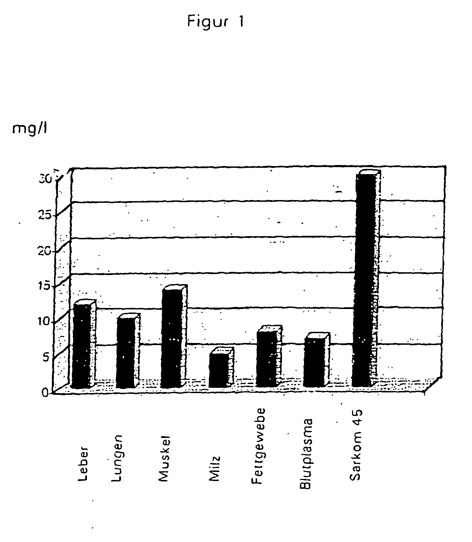

Die Verwendung des Mittels gemäß der Erfindung hat einen selektiven Schutz von gesundem Gewebe zur Folge, die Behandlung des malignen Tumors wird nicht beeinträchtigt. Eine isolierte Gabe der in der erfindungsgemäß verwendeten Vitamin-Kombination enthaltenen einzelnen Vitamin-Komponenten führt hingegen nicht zu einem selektiven Schutz bei Antitumortherapien. Beispielsweise konnte anhand von Tierversuchen gezeigt werden, dass eine isolierte Gabe von Vitamin E zu einer Anreicherung dieses Vitamins in dem Tumor führt, da der Tumor eine "Vitamin E-Falle" ist. In Figur 1 sind die Tocopherol-Gehalte in bestimmten Organen von Ratten mit Sarkom 45 gegenübergestellt.

Durch die Anreicherung des Antioxidans Vitamin E im Tumor der Ratten wird die Resistenz, also z.B. die Strahlenresistenz, des Tumors erhöht. Die Organe, die nicht-Tumor-befallen sind, weisen dagegen einen so geringen Vitamin E-Gehalt im Vergleich zum Sarkom 45 auf, dass diese nicht ausreichend vor Strahlung oder Cytostatika geschützt werden können.

Durch die erfindungsgemäße Verwendung der speziellen Vitamin-Kombination wird hingegen überraschenderweise selektiv gesundes Gewebe geschützt, es werden also die toxischen Erscheinungen von Strahlungstherapie und Cytostatika verringert. Dadurch wird die Antitumoreffektivität von Cytostatika und Bestrahlungen beträchtlich erhöht, was zur Verbesserung der Ergebnisse von Chemotherapie und Bestrahlungen bei Patienten mit malignen Tumoren führt.

Ein weiterer Vorteil der erfindungsgemäßen Verwendung der Vitamin-Kombination besteht darin, dass aufgrund der verringerten Nebenwirkungen von Antitumortherapien intensive Bestrahlungen und Chemotherapien möglich werden, die ohne den erfindungsgemäßen Schutz für die Patienten nicht verträglich wären.

Bei der Durchführung von Chemo- oder/und Strahlungstherapien mit dem erfindungsgemäßen Schutz vor Nebenwirkungen treten bei den betroffenen Patienten im Vergleich zu Patienten, die nicht gemäß der Erfindung geschützt waren, wesentlich weniger Lokalrezidive und Fernmetastasen auf. Die 5-jährige Überlebensrate und die durchschnittliche Lebenszeit solcher Patienten wird durch die erfindungsgemäße Verabreichung der Vitamin C, E, A-Kombination überraschenderweise beträchtlich gesteigert. Durch die Erfindung können Patienten mit malignen Tumoren also auch langanhaltend vor den toxischen Auswirkungen von Antitumorbehandlungen geschützt werden.

Im Gegensatz zu herkömmlichen Strahlenschutzmitteln wie Mexamin ist das erfindungsgemäß verwendete Vitamin-Mittel untoxisch. Mexamin besitzt neben der enormen Toxizität den Nachteil, dass es keine dauerhafte Strahlen-schützende Wirkung aufweist, da es eine kurze Wirkungsdauer hat.

Noch ein weiterer Vorteil der erfindungsgemäßen Verwendung der Vitamin-Kombination besteht darin, dass der Vitaminhaushalt der Tumorpatienten langanhaltend reguliert wird. Normalerweise treten bei Tumorpatienten selbst nach erfolgter chirurgischer Entfernung des malignen Tumors Hypovitaminosen auf. Eine Normalisierung des Vitaminhaushaltes führt zur Stabilisierung des Immunsystems der Patienten, was wiederum die Heilungschancen und somit die durchschnittliche Lebenserwartung erhöht.

Die Erfindung zeichnet sich außerdem durch die gute Verfügbarkeit der Wirkstoffe sowie deren einfache Handhabung aus.

Die Erfindung soll durch die Figuren und Beispiele näher erläutert werden.

Figurenbeschreibung

Figur 1: Gegenüberstellung der Tocopherol-Gehalte in bestimmten Organen von Ratten mit Sarkom 45.
Figur 2: MDA-Gehalt bei Ratten mit Sarkom 45 im Vergleich zu gesunden Tieren mit und ohne Verabreichung der erfindungsgemäßen Kombination.
Figur 3: Gewichte der Kroker-Sarkome von Tieren verschiedener Versuchs-Gruppen:

Gruppe I: Verabreichung der erfindungsgemäßen Vitamin-Kombination vor der Cyclophosphamid-Einnahme.

Gruppe II: Nur Cyclophosphamid-Einnahme.
Gruppe III: Nur erfindungsgemäße Vitamin-Konmbination.
Gruppe IV: Placebo.

Figur 4:  Häufigkeit der Fernmetastasenbildung bei den verschiedenen Gruppen I-IV (siehe auch Tabelle B4.2)
Figur 5:  Graphische Darstellung der durchschnittlichen Lebensdauer der Patienten aus den verschiedenen Gruppen (in Monaten).

Experimentelle und klinische Untersuchungen

A. Experimentelle Untersuchungen

1. Abhängigkeit der Wirksamkeit von der Dosis

Die Wirksamkeit der Kombination der Vitamine C, E und A ist abhängig von der Dosierung. Versuche hierzu wurden mit drei Monate alten weißen männlichen und weiblichen Ratten mit einem Gewicht von 150 bis 180 g durchgeführt, die einen transplantierten Sarkom 45 im Stadium intensiver Tumorproliferation trugen. Innerhalb von 4 bis 5 Tagen vor ihrer Dekapitation wurde ihnen die erfindungsgemäße Vitamin-Kombination verabreicht (Gruppe Ia). Die Gruppe Ib besteht aus vergleichbaren Ratten mit transplantiertem Sarkom 45, denen die Vitamin-Kombination nicht verabreicht wurde. Zum Vergleich dient außerdem Gruppe Ic, die aus gesunden Tieren besteht.
Einer anderen Versuchsratten-Gruppe (IIa) wurde entsprechend der Gruppe la die Kombination der Vitamine C, E und A verabreicht, wobei aber die Dosierung um das 10-fache verringert war. Die Gruppen IIb und IIc sind wiederum Ratten mit Sarkom 45 bzw. gesunde Tiere, denen die Vitamin-Kombination nicht verabreicht wurde.
In Tabelle A1.1 sind die verwendeten Dosen in mg pro Gramm Körpergewicht zusammengefasst. Die Vitamine wurden den Tieren subkutan injiziert.

Tabelle A1.1

| Gruppe | Ascorbinsäure | $\alpha$-Tocopherol-Acetat | Retinol-Acetat |
|---|---|---|---|
| Ia (Sarkom 45) | 0,2-0,3 mg | 0,08-0,09 mg | 0,001-0,002 mg |
| Ib (Sarkom 45) | - | - | - |
| Ic (gesund) | - | - | - |
| IIa (Sarkom 45) | 0,02-0,03 mg | 0,008-0,009 mg | 0,0001-0,0002 mg |
| IIb (Sarkom 45) | - | - | - |
| IIc (gesund) | - | - | - |

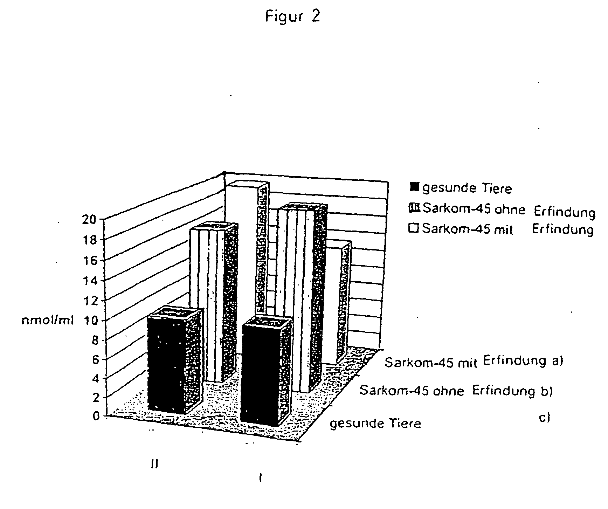

Die Tiere wurden getötet und der Malondialdehyd (MDA)-Gehalt in den Lebern der Tiere wurde untersucht. Malondialdehyd ist ein Produkt der Lipidperoxidation, welche durch die Tumorproliferation verstärkt ist. Der Gehalt an MDA in der Leber der untersuchten Gruppen gibt an, welches Ausmaß die Lipidperoxidation jeweils annimmt.
Aus Figur 2 ist ersichtlich, dass der MDA-Gehalt bei Ratten mit Sarkom 45 im Vergleich zu gesunden Tieren beträchtlich erhöht ist, da bei diesen Tieren verstärkt die Lipidperoxidation auftritt. Die Verminderung des MDA-Gehalts, also der Lipidperoxidation, durch die Vitamin C, E, A-Kombination ist, wie es aus Figur 2 ersichtlich ist, Dosis-abhängig. Der MDA-Gehalt in der Leber von Ratten der Gruppe la, denen also die Vitamin C, E, A-Kombination verabreicht worden war, ist im Vergleich zu Sarkom 45-Ratten, die die spezielle Vitamin-Kombination nicht erhalten hatten (Gruppe Ib) beträchtlich verringert und annähernd so niedrig, wie der MDA-Gehalt gesunder Tiere (Gruppe Ic). Im Gegensatz dazu erzielt man mit der um das 10-fache verringerten Dosis der Vitamin C, E, A-Kombination keinen antioxidativen Effekt; der MDA-Gehalt in der Leber der Tiere der Gruppe IIa ist sogar leicht erhöht gegenüber dem Wert, der in der Leber von Sarkom 45-Ratten gefunden wurde, denen die Vitamin-Kombination nicht verabreicht worden war (Gruppe IIb).

2. Schutz durch die Vitamin-Kombination bei der Bestrahlung

Drei Gruppen mit jeweils weißen rasselosen maskulinen Mäusen mit einem Gewicht von 18 bis 20 g wurde 24 Stunden vor der entsprechenden Ganzkörperbestrahlung mit der Gammastrahlen-Einrichtung "Rokus" (Kobalt-60) die erfindungsgemäße Vitamin-Kombination verabreicht. Die Vitamine A und E wurden den Tieren in öliger Lösung durch eine Schlundsonde zugeführt, Vitamin C wurde als wässrige Lösung subkutan injiziert. Die Dosis betrug (in mg pro Gramm Körpergewicht) 0,2-0,3 mg Ascorbinsäure, 0,08-0,09 $\alpha$-Tocopherol-Acetat und 0,001-0,002 Retinol-Acetat. Die Mäuse wurden mit 8 Gy (Gruppe Ia), 12 Gy (Gruppe Ib) bzw. 14 Gy (Gruppe Ic) bestrahlt, und es wurde beobachtet, welche Überlebenszeiten die Tiere aufwiesen. Zum Vergleich wurden drei Gruppen mit entsprechenden Mäusen bestrahlt, denen jedoch die Vitamin-Kombination nicht verabreicht worden war (Gruppen IIa, IIb und IIc).

In Tabelle A2.1 sind die Ergebnisse der Bestrahlungen zusammengefasst.

Tabelle A2.1

| Versuchsgruppe | % verendete Tiere/Tage bei Bestrahlungsdosis | | |
|---|---|---|---|
| | 8 Gy (a) | 12 Gy (b) | 14 Gy (c) |
| I (erfindungsgemäßer Schutz) | 16/30 | 50/9 | 50/8 |
| II (ohne Schutz) | 26/30 | 50/7 | 50/5 |

Aus der Tabelle A2.1 ist ersichtlich, dass nach 30 Tagen nach der Bestrahlung mit einer Dosis von 8 Gy nur 16 % der Tiere aus der Gruppe I verendet waren, wohingegen zur gleichen Zeit 26 % der Tiere aus der Gruppe II, denen die Vitamin-Kombination nicht verabreicht worden war, tot waren. Bei Erhöhung der Bestrahlungsdosis auf 12 Gy verblieben nach 9 Tagen 50 % der Tiere aus der Gruppe I, von den Tieren der Gruppe II sind bereits nach 7 Tagen 50 % verendet. Noch deutlicher wird die Schutzwirkung der Vitamin-Kombination, wenn mit einer Dosis von 14 Gy bestrahlt wird: Aus Gruppe I leben nach 8 Tagen noch 50 % der Tiere, wohingegen bei Gruppe II bereits nach 5 Tagen die Hälfte der Mäuse verendet ist.

Dementsprechend ist die durchschnittliche Lebensdauer der Mäuse deutlich höher, wenn ihnen vor der Bestrahlung die Vitamin-Kombination gemäß der Erfindung verabreicht wird (Tabelle A2.2).

Tabelle A2.2

| Versuchsgruppe | Durchschnittliche Lebensdauer (Tage) bei Bestrahlungsdosis | |
|---|---|---|
| | 12 Gy (b) | 14 Gy (c) |
| I (erfindungsgemäßer Schutz) | $9,0 \pm 0,6$* | $7,9 \pm 0,7$** |
| II (ohne Schutz) | $7,5 \pm 0,5$ | $5,5 \pm 0,3$ |
| *: -p < 0,05 <br> **: -p < 0,01 | | |

Bei einer Bestrahlungsdosis von 12 Gy können die Mäuse im Durchschnitt 9 Tage, bei 14 Gy 7,9 Tage überleben, wenn ihnen die Vitamin-Kombination verabreicht wurde. Tiere aus der Gruppe II überleben nur 7,5 bzw. 5,5 Tage.

Zum Vergleich des erfindungsgemäßen Strahlenschutzes mit der Wirkung herkömmlicher Strahlenschutzmittel wurden zum einen weiße rasselose männliche und weibliche Ratten mit einem Gewicht von 150 bis 180 g (Alter: 3 Monate) einer Ganzkörper-Gamma-Bestrahlung mit einer Dosierung von 11,4 Gy unterworfen, wobei die Tiere erfindungsgemäß durch die Verabreichung der Vitamin-Kombination mit der oben genannten Dosis 24 Stunden vor der Bestrahlung geschützt waren (Gruppe I). Zum anderen wurde eine andere Gruppe vergleichbarer Ratten genauso bestrahlt, wobei ihnen 24 Stunden vor der Bestrahlung das herkömmliche Strahlenschutzpräparat Mexamin verabreicht worden war (Gruppe II). Eine dritte Gruppe Ratten wurde ohne die Verabreichung eines Strahlenschutzmittels bestrahlt (Gruppe III). Die Überlebensrate und durchschnittliche Lebensdauer der Ratten aus den Gruppen I, II bzw. III sind in der Tabelle A2.3 zusammengefasst.

Tabelle A2.3

| Versuchsgruppe | Bestrahlungsdosis 11,4 Gy | |
|---|---|---|
| | % verendete Tiere/Tage | durchschnittliche Lebensdauer (Tage) |
| I (erfindungsgemäßer Schutz) | 50/8 <br> 100/16 | $8,4 \pm 4$* |
| II (Mexamin) | 50/8 <br> 100/13 | $8,2 \pm 4$* |
| III (ohne Schutz) | 50/5 | $5,4 \pm 0,3$ |

EP 1 492 524 B1

Tabelle fortgesetzt

| Versuchsgruppe | Bestrahlungsdosis 11,4 Gy | |
|---|---|---|
| | % verendete Tiere/Tage | durchschnittliche Lebensdauer (Tage) |
| | 100/9 | |
| *: -p < 0,05 | | |

Aus Tabelle A2.3 geht hervor, dass durch die Vitamin-Kombination eine beträchtliche Strahlenschutzwirkung erreicht wird, die zu einer Erhöhung der durchschnittlichen Lebensdauer der Tiere im Vergleich zur ungeschützten Bestrahlung bzw. der Bestrahlung bei Verabreichung von Mexamin führt. Die Ratten der Gruppe I haben eine durchschnittliche Lebensdauer von 8,4 Tagen, wohingegen die Tiere der Gruppe II, denen Mexamin anstatt des erfindungsgemäßen Vitamin-Mittels verabreicht wurde, nur im Durchschnitt 8,2 Tage nach der Bestrahlung leben. Ohne die Verabreichung eines Strahlenschutzmittels leben die Tiere im Durchschnitt 5,4 Tage (Gruppe III).

3. Schutz durch die Vitamin-Kombination bei der Chemotherapie

Zur Beurteilung der Schutzwirkung des erfindungsgemäßen Mittels wurde einer Gruppe von weißen rasselosen Ratten mit einem Gewicht von 150 bis 180 g (Alter: 3 Monate) 24 Stunden vor der Injektion von 5-Fluoruracil die Vitamin-Kombination (Dosis siehe 2) subkutan injiziert. 5-Fluoruracil wurde in einer Dosis von 1,8 mg pro kg Körpergewicht verabreicht (Gruppe I). Einer weiteren Gruppe Ratten wurde bei entsprechender 5-Fluoruracil-Behandlung die Vitamin-Kombination nicht zugeführt. In Tabelle A3.1 sind die Überlebensfähigkeiten der Ratten dieser beiden Gruppen gegenübergestellt.

Tabelle A3.1

| Versuchsgruppe | 5-Fluoruracil (1,8 mg pro kg Körpergewicht) Überlebensfähigkeit nach 8 Tagen [%] (p < 0,002) |
|---|---|
| I (erfindungsgemäßer Schutz) | $82 \pm 4,3$ % |
| II (ohne Schutz) | $61 \pm 5,4$ % |

Aus Tabelle A3.1 ist ersichtlich, dass nach 8 Tagen Beobachtungszeit 82 % der Ratten aus der Gruppe I, aber nur 61 % der Ratten aus der Gruppe II (ohne Schutz) überlebt hatten.

Die erfindungsgemäße Kombination aus den Vitaminen C, E und A zeigt also sowohl bei der Bestrahlungs- als auch bei der Chemotherapie eine herausragende Schutzwirkung, die so nicht zu erwarten war.

4. Schutz durch die Vitamin-Kombination bei einer kombinierten Strahlungs-/Chemotherapie

Eine Gruppe weißer rasseloser Ratten mit einem Gewicht von 150 bis 180 g wurde zunächst einer Ganzkörperbestrahlung (Gammastrahleneinrichtung siehe unter 2) mit einer Dosis von 8 Gy unterworfen, und dann wurde ihnen 5-Fluoruracil (Dosis: 1,8 mg pro kg Körpergewicht der Ratte) injiziert, wobei 24 Stunden vor der Bestrahlung die erfindungsgemäße Vitamin-Kombination in der unter 2. genannten Dosierung verabreicht worden war (Gruppe I). Eine zweite Gruppe Ratten wurde entsprechend behandelt, den Tieren wurde jedoch das Vitamin-Mittel zum Schutz nicht verabreicht (Gruppe II). Die Überlebensfähigkeit der Tiere der beiden Gruppen ist in Tabelle A4.1 gegenübergestellt.

Tabelle A4.1

| Versuchsgruppe | Bestrahlung (8 Gy) + 5-Fluoruracil (1,8 mg pro kg Körpergewicht) Überlebensfähigkeit nach 60 Tagen [%] |
|---|---|
| I (erfindungsgemäßer Schutz) | 73 |
| II (ohne Schutz) | 41 |

Aus Tabelle A4.1 geht hervor, dass auch bei einer kombinierten Chemo/Strahlungs-Therapie die Überlebensfähigkeit der Versuchstiere durch die Vitamin C, E, A-Kombination gemäß der Erfindung immens erhöht werden kann. So überleben die Tiere der Gruppe I nach 60 Tagen zu 73 %, wohingegen nur 41 % der Tiere aus der Gruppe II nach 60 Tagen noch leben.

5. Selektivität der Vitamin C, E, A-Kombination

Der Strahlungsschutz bzw. der Schutz bei Chemotherapie der Vitamin-Kombination gemäß der Erfindung wirkt selektiv auf gesundes Gewebe und vermindert nicht die Wirkung der Bestrahlung bzw. des Cytostatikums auf den Tumor. Diese Selektivität wurde experimentell bestätigt, indem 4 Ratten der oben genannten Spezies mit Karzino-Sarkom Walker einer einmaligen örtlichen Bestrahlung ausgesetzt wurden, wobei nur der Tumor mit einer Dosis von 20 Gy bestrahlt wurde. Dabei wurde die Röntgeneinrichtung RUM-11 verwendet. Zwei dieser Ratten wurde 24 Stunden vor der Bestrahlung die Vitamin-Kombination (Dosis siehe unter 2.) subkutan verabreicht. Gleich nach der Bestrahlung wurde eine Transplantation der Tumoren der bestrahlten Spender auf gesunde Tiere ausgeführt. Die Empfänger-Ratten bekamen das Vitamin-Mittel nicht verabreicht. Es wurden die Tumor-Volumina aller untersuchten Tiergruppen bestimmt. Die Ergebnisse zeigten, dass keine Unterschiede in den Veränderungen der Tumorvolumina vorlagen. Die erfindungsgemäße Vitamin-Kombination besitzt somit keinen Strahlenschutzeffekt bezüglich des malignen Tumors und kann selektiv zum Schutz von gesundem Gewebe bei Bestrahlungen eingesetzt werden.

Die aus dem obigen Experiment hervorgehende Selektivität des erfindungsgemäßen Mittels wurde auch bei der folgenden Untersuchung bestätigt:

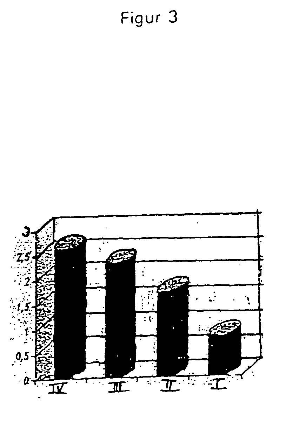

Weißen rasselosen Mäusen mit einem Gewicht von 20 bis 25 g wurde durch herkömmliche Verfahren ein Kroker-Sarkom implantiert. Einer Versuchstier-Gruppe wurde 24 Stunden vor der Einnahme von Cyclophosphamid (50 mg pro kg Körpergewicht) die Vitamin-Kombination verabreicht (Gruppe I). Zum Vergleich wurde eine Gruppe Mäuse entsprechend behandelt, jedoch ohne die Verabreichung des erfindungsgemäßen Vitamin-Mittels (Gruppe II). Der Tiergruppe III wurde nur die Vitamin-Kombination verabreicht, die Kontrollgruppe IV erhielt einen Placebo. Die Figur 3 stellt das Gewicht der Kroker-Sarkome der Tiere aus den verschiedenen Versuchsgruppen gegenüber. Die Vitamine wurden in einer Dosis von 300 mg (Vitamin C), 90 mg (Vitamin E) und 20 mg (Vitamin A), jeweils pro kg Körpergewicht, verabreicht.

Die Ergebnisse sind in der Figur 3 veranschaulicht. Daraus geht hervor, dass die Verabreichung des Vitamin-Mittels gemäß der Erfindung den Tumor nicht vor dem Cytostatikum Cyclophosphamid schützt. Dies ist ersichtlich, da das Gewicht des Tumors sogar geringer ist als bei einer alleinigen Verabreichung von Cyclophosphamid. Die erfindungsgemäße Verabreichung der Vitamin-Kombination ohne die Gabe eines Cytostatikums führt auch im Vergleich zur Verabreichung eines Placebos zu einer Verringerung des Tumor-Gewichts.

B. Klinische Untersuchungen

1. Charakterisierung der Patienten

Wenn es in den Untersuchungen nicht anders angegeben ist, war das Verhältnis Männer zu Frauen in den jeweiligen Behandlungsgruppen praktisch gleich, und die meisten Patienten waren im Alter von 51 bis 60 Jahren. Sie hatten ein Körpergewicht von 50 - 100 kg.

2. Schutz durch die Vitamin-Kombination bei chirurgischen Eingriffen

Einer Gruppe von Patienten mit Magenkrebs im Frühstadium wurde in der Voroperationsphase innerhalb von 7 Tagen täglich die Vitamin C, E, A-Kombination verabreicht. Danach wurde ein chirurgischer Eingriff durchgeführt und die operierten Patienten erhielten über 1,5 Jahre hinweg die Vitamin-Kombination (Gruppe I). Eine andere Gruppe vergleichbarer Patienten mit Magenkrebs im Frühstadium wurden entsprechend operiert, jedoch wurde ihnen nicht die erfindungsgemäße Vitamin-Kombination verabreicht (Gruppe II).

Die Vitamin-Kombination wurde mit einer Dosis von 2,0 g Ascorbinsäure, 0,6 g $\alpha$-Tocopherol-Acetat und 0,033 g Retinol-Acetat oral verabreicht.

In der Tabelle B2.1 sind die prozentualen Überlebensraten der Patienten beider Gruppe nach 1, 3 und 5 Jahren aufgeführt.

Tabelle B2.1

| Patientengruppe (Stadium 1B) | Überlebensrate (%) nach | | |
|---|---|---|---|
| | 1 Jahr | 3 Jahren | 5 Jahren |
| I (gemäß Erfindung) | 96,6 ± 3,2 | 96,6 ± 3,2 | 90,9 ± 6,3* |
| II (nur Operation) | 90,0 ± 4,7 | 77,5 ± 6,6 | 70,0 ± 6,8 |
| *: -p < 0,025 im Vergleich zu "nur Operation" | | | |

Aus der Tabelle B2.1 geht hervor, dass durch die Verabreichung der Vitamin C, E, A-Kombination die Überlebensrate im Vergleich zu der der Gruppe II erhöht war. Nach 1 Jahr überleben 5 % mehr aus der Gruppe I als Patienten der Gruppe II, nach 3 Jahren beträgt die Differenz bereits knapp 20 % und nach 5 Jahren über 20 %.

Auch bei Patienten mit Magenkrebs im fortgeschrittenen Stadium können durch die erfindungsgemäße Verabreichung der Vitamin-Kombination erstaunliche Überlebensraten der Patienten erreicht werden. Dies wird durch den Vergleich der prozentualen Überlebensraten der Patienten aus der Gruppe I, denen zusätzlich zur Operation die Vitamin-Kombination verabreicht wurde, mit den Patienten der Gruppe II, die nur operiert wurden, deutlich. Tabelle B2.2 stellt die Überlebensraten nach 1, 3 und 5 Jahren gegenüber.

Tabelle B2.2

| Patientengruppe (Stadium 3A) | Überlebensrate (%) nach | | |
|---|---|---|---|
| | 1 Jahr | 3 Jahren | 5 Jahren |
| I (gemäß Erfindung) | 85,7 ± 9,3 | 53,0 ± 14,0 | 53,0 ± 14,0* |
| II (nur Operation) | 84,7 ± 8,1 | 23,5 ± 7,2 | 11,7 ± 5,5 |
| *: -p < 0,01 im Vergleich zu "nur Operation" | | | |

Insbesondere nach 3 und 5 Jahren ist die Überlebensrate der Patienten aus der Gruppe I beträchtlich gegenüber der der Patienten aus Gruppe II erhöht; nach 3 Jahren um 30 %, nach 5 Jahren um über 40 %.

3. Schutz durch die Vitamin-Kombination bei der Chemotherapie

a)

Einer Gruppe I mit Patienten mit Magenkrebs im Stadium IV (13 Patienten) wurde über 7 Tage täglich 750 mg 5-Fluoruracil intravenös verabreicht. Die Pausen zwischen den Therapiezyklen dauerten 2 bis 2,5 Monate. Während der gesamten Chemotherapie wurde diesen Patienten täglich die Vitamin-Kombination mit einer täglichen Dosis, wie sie unter 2. angegeben ist, verabreicht. Mit einer Gruppe II aus Patienten im gleichen Krankheitsstadium wurde eine entsprechende Therapie mit 5-Fluoruracil durchgeführt, jedoch ohne den Schutz durch das Vitamin-Mittel.

Die Ergebnisse der Kontrollgruppen sind älteren Literaturstellen entnommen.

In Tabelle B3.1 sind die Überlebensraten der Patienten der Gruppen I und II gegenübergestellt.

Tabelle B3.1

| Patientengruppe (Stadium IV) | Überlebensrate (%) nach (Monate) | | | | | | |
|---|---|---|---|---|---|---|---|
| | 3 | 6 | 9 | 12 | 18 | 22 | 44 |
| I (5-Fu + Erfindung) | 100 | 77.0 | 53,9 | 46,2 | 30,8 | 30,8 | 23,1 |
| II (nur 5-Fu) | 86,7 | 66,7 | 40,0 | 20,0 | 13,7 | 0 | - |

Der Tabelle B3.1 kann entnommen werden, dass die Überlebensrate der Patienten mit Magenkrebs im Stadium IV beträchtlich erhöht wird, wenn ihnen während der Chemotherapie die Vitamin-Kombination verabreicht wird. Nach 44 Monaten leben noch 23,1 % der Patienten aus Gruppe I. Von Gruppe II überlebt jedoch kein Patient bis zum 22. Monat. Die Überlebensrate der Gruppe I nach 12 bzw. 18 Monaten ist mehr als doppelt so hoch als die der Gruppe II. Durch die Erfindung ist es also möglich, die Effektivität der Chemotherapie beträchtlich zu steigern und so ein längeres Überleben der Patienten zu gewährleisten.

Die Ergebnisse der durchgeführten Gruppenuntersuchungen zeigen, dass die Vitamin-Kombination der Erfindung die Antitumoreffektivität von Chemotherapien erhöht und die toxische Wirkung im Organismus beträchtlich reduziert, was zur Verbesserung der Chemotherapie-Ergebnisse bei Patienten mit Magenkrebs im fortgeschrittenen Stadium führt.

b)

Bei einer 74-jährigen Patientin wurde im Juni 2002 ein Magenkarzinom mit initialer Hautmetastase diagnostiziert. Histologisch handelte es sich um ein gering differenziertes Adeno-Karzinom, passend zum diffusen Typ nach Lauren. Im August 2002 erfolgte eine Laparotomie, es wurde eine Peritonealkarzinose gefunden, wobei der Befund inoperabel war. Es wurden lediglich Probebiopsien entnommen und ein Port-System zur Verabfolgung von Chemotherapie implantiert. Es wurde über 5 Tage eine 5-FU Dauerinfusion in 4-wöchigen Abständen mit insgesamt 4 Zyklen durchgeführt, die infundierte Dosis betrug 600 mg/m$^2$ KO (Körperoberfläche) täglich. Daran anschließend erfolgte die Applikation von zwei weiteren Zyklen mit einer Dosis von 750 mg/m$^2$ KO täglich, ebenfalls über jeweils 5 Tage. Während der gesamten Zeitdauer der Chemotherapie wurde der Vitaminkomplex täglich oral in folgender Dosierung eingenommen: Vitamin C 2000 mg, Vitamin E 600 mg und Vitamin A 33 mg. Das Befinden der Patientin war während dieser Zeit hervorragend, es konnten weder Leukozytenabfälle noch Übelkeit, Erbrechen oder Mukositis festgestellt werden. Bildgebende und blutchemische Kontrolluntersuchungen ergaben, dass die Erkrankung unter der kombinierten Therapie von 5-FU mit der erfindungsgemäßen Vitamin-Kombination absolut stabil war.

Dieses Behandlungsresultat zeigt, dass mittels der erfindungsgemäßen Vitamin-Kombination die Verträglichkeit von Chemotherapie beträchtlich erhöht wird, wobei gleichzeitig ein verbesserter Therapieerfolg beobachtet werden kann. Durch die vorliegende Erfindung wird also die Möglichkeit gegeben, selbst bei schweren Krebserkrankungen die Behandlungschancen und somit die Lebenserwartung der betroffenen Patienten zu verbessern.

4. Schutz durch die Vitamin-Kombination bei Strahlungstherapien

Ein großes Problem bei Tumor-Patienten besteht darin, dass diese Patienten in der post-operativen Phase meist einen Rückfall erleiden und sich Fernmetastasen ausbilden. Dies tritt bei 20 bis 50 % der Patienten ein.

Patienten mit Adenokarzinomen in verschiedenen Stadien wurden verschiedenen Therapien unterzogen. Gruppe I wurde in der voroperativen Phase einer Strahlungstherapie mit einer Dosis von 20 Gy ausgesetzt, wobei den Patienten die erfindungsgemäße Vitamin-Kombination verabreicht worden war. Danach erfolgte die Operation. Die Patienten der Gruppe II wurden analog der Patienten aus Gruppe I behandelt, jedoch betrug die Strahlungsdosis 30 Gy. Patienten aus der Gruppe III wurden vor der Operation einer Strahlungstherapie mit einer Dosis von 20 Gy unterworfen, wobei ihnen jedoch nicht die Vitamin-Kombination verabreicht worden war. Gruppe IV bestand aus Patienten, die nur operiert wurden.

In der Tabelle B4.1 ist die Häufigkeit der Fernmetastasenbildung bei Patienten aus den Gruppen I bis IV je nach deren Adenokarzinom-Struktur nach einem Beobachtungszeitraum von 5 Jahren aufgeführt.

Tabelle B4.1

| Adenokarzinomstadium | Gruppe I | | Gruppe II | | Gruppe III | | Gruppe IV | |
|---|---|---|---|---|---|---|---|---|
| | $\frac{A}{B}$ * | in % | $\frac{A}{B}$ * | in % | $\frac{A}{B}$ * | in % | $\frac{A}{B}$ * | in % |
| hoch differenziert | $\frac{0}{1}$ | 0,0 | $\frac{0}{11}$ | 0,0 | $\frac{2}{6}$ | 33,3 | $\frac{6}{32}$ | 18.8 |
| mäßig differenziert | $\frac{5}{19}$ | 26,3 | $\frac{2}{26}$ | 7,7 | $\frac{2}{10}$ | 20,0 | $\frac{9}{43}$ | 20,9 |
| wenig differenziert | $\frac{0}{3}$ | 0,0 | $\frac{0}{4}$ | 0,0 | $\frac{2}{2}$ | 100 | $\frac{0}{9}$ | 0,0 |
| * A = Zahl der Patienten mit Fernmetastasen B = Gesamtzahl der Patienten | | | | | | | | |

Am häufigsten wurde die Fernmetastasenbildung eines hochdifferenzierten Adenokarzinoms in der Gruppe III registriert: 33,3 % der Patienten dieser Gruppe wiesen nach einem Beobachtungszeitraum von 5 Jahren Fernmetastasen auf. Dieser Wert ist viel höher als bei den Patienten, die nur einer Operation unterworfen wurden (Gruppe IV, 18,8 %). Die Strahlungstherapie hat also einen schädigenden Einfluss auf den Organismus der Patienten, es sei denn, den Patienten wird zum Schutz erfindungsgemäß die Vitamin-Kombination verabreicht. So wurden bei Patienten der Gruppe I, die ebenfalls mit 20 Gy bestrahlt wurden, jedoch durch die erfindungsgemäße Vitamin-Kombination geschützt waren, keine Fernmetastasen festgestellt. Selbst bei Erhöhung der Strahlung auf 30 Gy (Gruppe II), wiesen die Patienten keine Fernmetastasen auf. Auch bei niedrig differenziertem Adenokarzinom ist dieser Effekt beträchtlich: Alle Patienten, also 100 % der Gruppe III, weisen Fernmetastasen auf, wohingegen weder bei der Gruppe I noch bei der Gruppe II Fernmetastasen beobachtet wurden.

Auch bei Patienten mit Dickdarmkrebs in verschiedenen Stadien ist die Schutz-Wirkung des Vitamin-Mittels bei verschiedenen Strahlungstherapien zu beobachten. In Tabelle B4.2 ist die Häufigkeit der Fernmetastasenbildung bei Patienten mit Dickdarmkrebs in verschiedenen Stadien nach einem Beobachtungszeitraum von 5 Jahren aufgeführt.

EP 1 492 524 B1

Die Patienten der Gruppe I bis IV wurden den oben genannten Therapien unterzogen.

Tabelle B4.2

| Dickdarmkrebs-Stadium | Gruppe I | | Gruppe II | | Gruppe III | | Gruppe IV | |
|---|---|---|---|---|---|---|---|---|
| | $\frac{A}{B}$ | in % | $\frac{A^{\bullet}}{B}$ | in % | $\frac{A^{\bullet}}{B}$ | in % | $\frac{A^{\bullet}}{B}$ | in % |
| T3-4 NO-MO (II) | $\frac{2}{16}$ | 12,5 | $\frac{1}{20}$ | 5,0 | $\frac{2}{11}$ | 18,2 | $\frac{8}{55}$ | 14,5 |
| T2-4N1-MO (III) | $\frac{2}{7}$ | 28,6 | $\frac{1}{12}$ | 8,3 | $\frac{4}{8}$ | 50,0 | $\frac{8}{17}$ | 47,1 |
| * A = Zahl der Patienten mit Fernmetastasen<br>B = Gesamtzahl der Patienten | | | | | | | | |

Besonders deutlich wird aus Tabelle B4.2, dass die erfindungsgemäße Vitamin-Kombination die Effektivität der Strahlungstherapie steigert, da die Fernmetastasenbildung der Patienten der Gruppen I und II im Vergleich zu nur bestrahlten, nicht erfindungsgemäß geschützten Patienten, verringert wird. Die Strahlungsdosis kann somit erhöht werden, ohne dass gesundes Gewebe der Patienten geschädigt wird. Patienten der Gruppe II, die mit 30 Gy bestrahlt wurden, wiesen nur zu 5 % (T3-4NO-MO (II)) bzw. 8,3 % (T2-4N 1-MO(III)) Fernmetastasen auf, wohingegen Patienten der Gruppe III, die nur mit 20 Gy bestrahlt worden waren und keine Vitamin-Kombination erhalten hatten, zu 18,2 % bzw. 50,0 % Fernmetastasen aufwiesen.

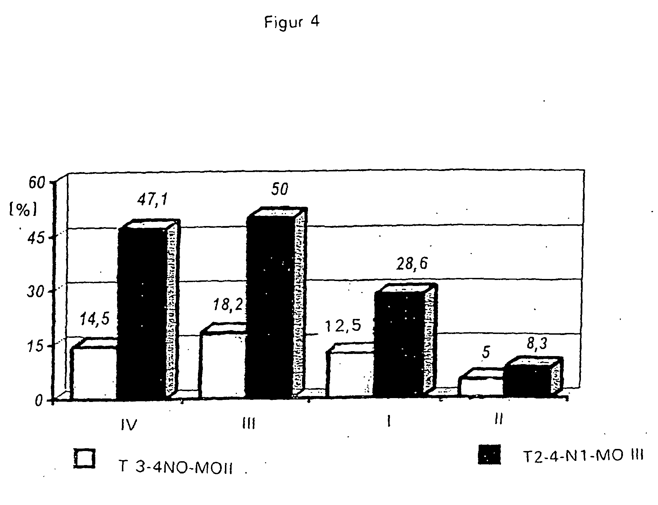

Diese Ergebnisse sind in Figur 4 graphisch veranschaulicht.

Die gesamte Häufigkeit der Fernmetastasenbildung je nach Behandlungsmethode (Gruppen I bis IV) von Patienten mit Dickdarmkrebs nach einem Beobachtungszeitraum von 5 Jahren ist in Tabelle B4.3 zu sehen.

Tabelle B4.3

| | Gruppe I | | Gruppe II | | Gruppe III | | Gruppe IV | |
|---|---|---|---|---|---|---|---|---|
| | $\frac{A^{\bullet}}{B}$ | in % | $\frac{A^{\bullet}}{B}$ | in % | $\frac{A^{\bullet}}{B}$ | in % | $\frac{A}{B}$ | in % |
| Anzahl Patienten mit Fernmetastasenbildung | $\frac{5}{23}$ | 21,7 $\pm$ 1,9 | $\frac{2}{41}$ | 4,9 $\pm$ 1,5** | $\frac{6}{20}$ | 30,0 $\pm$ 4,5 | $\frac{16}{87}$ | 18,4 $\pm$ 3,4 |
| * A = Zahl der Patienten mit Fernmetastasen<br>B = Gesamtzahl der Patienten<br>**p < 0,05 im Vergleich zu Gruppe III | | | | | | | | |

Am häufigsten tritt Fernmetastasenbildung bei der Bestrahlung der Patienten auf (30 %, Gruppe III). Die erfindungsgemäße Vitamin-Kombination kann das gesunde Gewebe effektiv gegen Strahlung schützen. Nur 21,7 % der Patienten aus Gruppe I, die unter dem erfindungsgemäßen Schutz entsprechend bestrahlt wurden, weisen Fernmetastasen auf. Besonders deutlich wird der Schutzeffekt dadurch, dass bei höherer Strahlungsdosis (30 Gy, Gruppe II), dieser Wert sogar wesentlich niedriger ist (4,9 %).

Die Anwendung der Vitamin-Kombination bei der präoperativen Strahlungstherapie führt zu einer beträchtlichen Senkung der Schädigung von gesundem Gewebe. Durch den erfindungsgemäßen Schutz ist es möglich, bei höheren Dosen zu bestrahlen, und so die Effektivität der Therapien zu steigern.

5. Schutz durch die Vitamin-Kombination bei kombinierten Chemo/Strahlungs-Therapien

a)

Verschiedene Patientengruppen wurden einer kombinierten Chemo/Strahlungs-Therapie ausgesetzt. Die Patienten mit Pankreas-Krebs wurden chirurgisch behandelt und dann einer nachoperativen Strahlungstherapie mit anschließender Polychemotherapie ausgesetzt:

12

Gruppe I: Strahlungstherapie mit Gesamtfokusdosis 35 Gy (Einzelfokusdosis 7 Gy), 6 Therapiezyklen Polychemotherapie

Gruppe II: Strahlungstherapie als Sekrationskur mit Bestrahlungsdosen 28 Gy (Einzelfokusdosis 4 Gy) plus 30 Gy (Einzelfokusdosis 2 Gy), 6 Polychemotherapie-Therapiezyklen.

Die Polychemotherapie wurde 15 bis 20 Tage nach der Strahlungstherapie vorgenommen. Innerhalb der ersten 5 Tage wurde 5-Fluoruracil mit 300 mg/m$^2$, Doxorubicin wurde am ersten Tag mit 50 mg/m$^2$ und Cisplatin mit 20 mg/m$^2$ innerhalb der ersten 5 Tage verabreicht. Die Cytostatika wurde intravenös zugeführt. Die Pausen zwischen den Polychemotherapie-Therapiezyklen betrugen 25 bis 30 Tage. Allen Patienten der Gruppen I und II wurde 24 Stunden vor jeder Behandlung die Vitamin-Kombination einmal täglich oral gegeben. Als Vergleichsgruppe diente eine Gruppe Patienten, die nur chirurgisch behandelt wurden (Gruppe III). Die angegebenen Daten zur Kontrollgruppe III stammen aus Versuchen, die in älteren Literaturstellen zu finden sind.

In der Tabelle B5.1 sind die Überlebensraten der Patienten aus den Gruppen I bis III gegenübergestellt.

Tabelle B5.1

| Patientengruppe | Überlebensrate (%) nach | | | |
|---|---|---|---|---|
| | 1 Jahr | 2 Jahren | 3 Jahren | 5 Jahren |
| I | 30,0 | 5,0 | 0 | - |
| II | 45,0 | 15,0 | 10,0 | 5,0 |
| III (Kontrolle) | 0 | - | - | - |

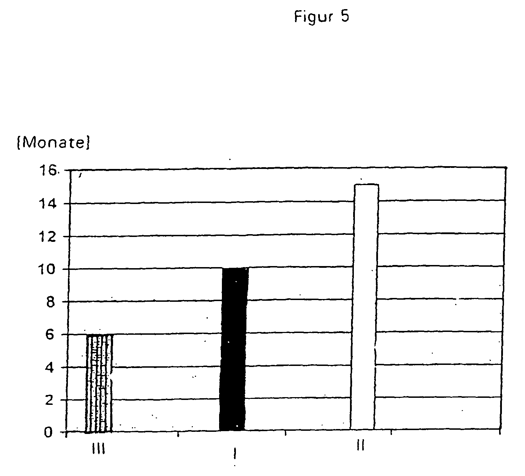

Alle Patienten der Kontrollgruppe III waren innerhalb von 12 Monaten verstorben. Dahingegen ermöglicht die kombinierte Chemo/Strahlungs-Therapie zusammen mit dem Vitamin-Mittel, dass auch nach 5 Jahren noch 5 % der Patienten der Gruppe II leben. Die mittlere Überlebenszeit der Patienten der drei Untersuchungsgruppen ist in Figur 5 graphisch dargestellt.

Ein Vergleich mit einer Gruppe, die der gleichen Chemo/Strahlungs-Therapie wie die Gruppen I und II, jedoch ohne den Schutz des Vitamin-Mittels unterworfen wurde, ist ethisch nicht vertretbar, jedoch konnten die Werte der Kontrollgruppen der Literatur entnommen werden. Sie zeigen, dass die Toxizität der verabreichten Cytostatika bzw. die Strahlungsdosen so gravierend sind, dass die Effektivität der Therapien eingeschränkt wird. Nur mit dem Schutz der erfindungsgemäßen Vitamin-Kombination ist es möglich, derart hohe Dosen anzuwenden, wodurch die Therapien außerordentlich erfolgreich sind.

b)

Bei einer 59-jährigen Patientin wurde im April 2002 ein inflammatorisches Mammakarzinom der linken Brust diagnostiziert. Histologisch fand sich ein gering differenziertes invasivduktales Mammakarzinom mit ausgedehnter Lymphangiosis carcinomatosa der Haut. Hormonrezeptorstatus: Östrogenrezeptor 60 %, Progesteronrezeptor 70 % positiv. Her2-Neu: Dako Score 1-2, damit grenzwertig. Neoadjuvant erfolgte die Gabe von 6 Zyklen Chemotherapie mit Epirubicin/Cyclophosphamid intravenös Tag 1 in 3-wöchigen Abständen. Bei vier Zyklen war die Dosis 90 mg/m$^2$ KO Epirubicin, 600 mg/m$^2$ KO Cyclophosphamid. Bei den beiden letzten Zyklen wurde die Epirubicindosis auf 120 mg/m$^2$ erhöht. Von September bis Oktober 2002 wurden die linke Brust und die entsprechenden Lymphabflusswege mit 50 Gy bestrahlt. Während der Bestrahlung erhielt die Patientin täglich die erfindungsgemäße Vitamin-Kombination mit Vitamin C 2000 mg, Vitamin E 600 mg und Vitamin A 33 mg. Die Strahlentherapie wurde hervorragend vertragen, es fanden sich nur eine geringgradige Rötung der bestrahlten Hautareale, Übelkeit und Erbrechen traten ebensowenig auf wie Leukozytenabfälle. Im Dezember 2002 wurden aus der linken Brust Stanzpräparate entnommen, die keinen Hinweis auf Malignität ergaben.

Dieser Behandlungsbericht veranschaulicht weiterhin eindrucksvoll die Verbesserung der Verträglichkeit einer Antitumortherapie aufgrund der Verabreichung der erfindungsgemäßen Vitamin-Kombination. Besonders bemerkenswert ist die Tatsache, dass trotz einer sehr hohen Bestrahlungsdosis von 50 Grey keine der sonst üblichen Nebenwirkungen wie Verschlechterung des Blutbilds, Übelkeit etc. beobachtet wurden. Im Allgemeinen sind derart hohe Strahlungsdosen mit gravierenden Nebenwirkungen verbunden. Durch die Verabreichung des erfindungsgemäßen Mittels wird folglich die Möglichkeit geschaffen, effektivere Chemo-/Strahlungstherapien anzuwenden und damit bessere Heilungserfolge bei malignen Tumoren zu erzielen. Dies wird beispielsweise durch den obigen Fall veranschaulicht, bei welchem nach erfindungsgemäßer Behandlung keine Malignität in den entnommenen Stanzpräparaten aus der linken Brust der Patientin mehr diagnostiziert wurde.

Zusammenfassend lässt sich sagen, dass durch die erfindungsgemäße Verabreichung der Vitamin C, E, A-Kombination die Verträglichkeit von Strahlungs- oder/und Chemo-Therapien beträchtlich erhöht wird, da gesundes Gewebe selektiv geschützt wird. So ist es möglich, auch sehr hohe Strahlungsdosen und Cytostatika-Dosen anzuwenden bzw. zu verabreichen.

**Patentansprüche**

1. Verwendung einer Kombination aus Ascorbinsäure, Tocopherol und Retinol, wobei Ascorbinsäure frei und Tocopherol und Retinol frei oder/und in Form ihrer pharmazeutisch annehmbaren Ester vorliegen und in einem Masseverhältnis zueinander von 1,5-2,5:0,3-0,8:0,028-0,033, bezogen auf Ascorbinsäure, $\alpha$-Tocopherol als Acetat und Retinol als Acetat, enthalten sind, zur Herstellung eines Mittels zur Verbesserung der Verträglichkeit von Chemotherapie oder/und Strahlungstherapie maligner Tumoren.

2. Verfahren zur Herstellung eines Mittels, das die Verträglichkeit von Chemotherapie oder/und Strahlungstherapie maligner Tumoren verbessert,
   **dadurch gekennzeichnet,**
   **dass** man eine Kombination aus Ascorbinsäure, Tocopherol und Retinol, wobei Ascorbinsäure frei, Tocopherol und Retinol frei oder/und in Form ihrer pharmazeutisch annehmbaren Ester, in einem Masseverhältniszueinander von 1,5-2,5:0,3-0,8:0,028-0,033, bezogen auf Ascorbinsäure, $\alpha$-Tocopherol als Acetat und Retinol als Acetat, vorliegen, als Wirkstoff zubereitet.

3. Verwendung nach Anspruch 1,
   **dadurch gekennzeichnet,**
   **dass** das Mittel Ascorbinsäure, Tocopherol und Retinol in einem Masseverhältnis zueinander von 1,8-2,1:0,5-0,65: 0,030-0,032, bezogen auf Ascorbinsäure, $\alpha$-Tocopherol als Acetat und Retinol als Acetat, enthält.

4. Verfahren nach Anspruch 2,
   **dadurch gekennzeichnet,**
   **dass** das Mittel Ascorbinsäure, Tocopherol und Retinol in einem Masseverhältnis zueinander von 1,8-2,1:0,5-0,65: 0,030-0,032, bezogen auf Ascorbinsäure, $\alpha$-Tocopherol als Acetat und Retinol als Acetat, enthält.

5. Verwendung nach Anspruch 1 oder 2,
   **dadurch gekennzeichnet,**
   **dass** Ascorbinsäure, Tocopherol und Retinol in einer Minimaldosis von 150 mg/Tag, 50 mg/Tag und 3 mg/Tag und in einer Maximaldosis von 2,5 g/Tag, 800 mg/Tag und 33 mg/Tag, jeweils bezogen auf Ascorbinsäure, $\alpha$-Tocopherol als Acetat und Retinol als Acetat, verabreicht werden.

6. Verwendung nach Anspruch 5,
   **dadurch gekennzeichnet,**
   **dass** die Minimaldosis 1,5 g/Tag, 300 mg/Tag und 28 mg/Tag, bezogen auf Ascorbinsäure, $\alpha$-Tocopherol als Acetat und Retinol als Acetat, verabreicht werden.

7. Verwendung nach einem der Ansprüche 1, 3, 5 und 6,
   **dadurch gekennzeichnet,**
   **dass** das Mittel $\alpha$-Tocopherol oder/und Retinol in Form ihrer pharmazeutisch annehmbaren Ester enthält.

8. Verwendung nach Anspruch 7,
   **dadurch gekennzeichnet,**
   **dass** das Mittel $\alpha$-Tocopherol oder/und Retinol in Form ihrer Essigsäureoder/und Bernsteinsäureester enthält.

9. Verwendung nach Anspruch 7,
   **dadurch gekennzeichnet,**
   **dass** das Mittel $\alpha$-Tocopherol und Retinol in Form ihrer Acetate enthält.

10. Verwendung nach einem der Ansprüche 1, 3 und 5 bis 9,
    **dadurch gekennzeichnet,**
    **dass** das Mittel ferner pharmazeutisch annehmbare Bindemittel, Geschmacksstoffe Farbstoffe, Süßungsmittel

EP 1 492 524 B1

oder/und andere für Pharmazeutika übliche Zusatzstoffe enthält.

11. Kombination aus Ascorbinsäure, Tocopherol und Retinol, wobei Ascorbinsäure frei und Tocopherol und Retinol frei oder/und in Form ihrer pharmazeutisch annehmbaren Ester vorliegen und in einem Masseverhältniszueinandervon 1,5-2,5:0,3-0,8:0,028-0,033, bezogen auf Ascorbinsäure, α-Tocopherol als Acetat und Retinol als Acetat, enthalten sind, zur Verwendung als Mittel zur Verbesserung der Verträglichkeit von Chemotherapie oder/und Strahlungstherapie maligner Tumoren.

12. Verwendung einer Kombination aus Ascorbinsäure, Tocopherol, Retinol und β-Carotin, wobei Ascorbinsäure frei und Tocopherol und Retinol frei oder/und in Form ihrer pharmazeutisch annehmbaren Ester und β-Carotin frei vorliegen und einem Masseverhältnis zueinander von 1,5-2,5:0,3-0,8:0,028-0,033, bezogen auf Ascorbinsäure, α-Tocopherol als Acetat und Retinol als Acetat zusammen mit β-Carotin enthalten sind, zur Herstellung eines Mittels zur Verbesserung der Verträglichkeit von Chemotherapie oder/und Strahlungstherapie maligner Tumoren.

13. Verfahren zur Herstellung eines Mittels, das die Verträglichkeit von Chemotherapie oder/und Strahlungstherapie maligner Tumoren verbessert,
**dadurch gekennzeichnet,**
**dass** man eine Kombination aus Ascorbinsäure, Tocopherol, Retinol und β-Carotin, wobei Ascorbinsäure frei, Tocopherol und Retinol frei oder/und in Form ihrer pharmazeutisch annehmbaren Ester und β-Carotin frei, in einem Masseverhältnis zueinander von 1,5-2,5:0,3-. 0,8:0,028-0,033, bezogen auf Ascorbinsäure, α-Tocopherol als Acetat und Retinol als Acetat zusammen mit β-Carotin vorliegen, als Wirkstoff zubereitet.

14. Verwendung nach Anspruch 12,
**dadurch gekennzeichnet,**
**dass** die Gesamtmenge an Retinol und β-Carotin aus 1/3 Retinol und 2/3 β-Carotin zusammengesetzt ist.

15. Verfahren nach Anspruch 13,
**dadurch gekennzeichnet,**
**dass** die Gesamtmenge an Retinol und β-Carotin aus 1/3 Retinol und 2/3 β-Carotin zusammengesetzt ist.

16. Verwendung nach Anspruch 12,
**dadurch gekennzeichnet,**
**dass** die Dosis β-Carotin 10 mg/Tag bis 25 mg/Tag beträgt.

17. Verfahren nach Anspruch 13,
**dadurch gekennzeichnet,**
**dass** die Dosis β-Carotin 10 mg/Tag bis 25 mg/Tag beträgt.

18. Kombination aus Ascorbinsäure, Tocopherol, Retinol und β-Carotin, wobei Ascorbinsäure frei und Tocopherol und Retinol frei oder/und in Form ihrer pharmazeutisch annehmbaren Ester und β-Carotin frei vorliegen und in einem Masseverhältnis zueinander von 1,5-2,5:0,3-0,8:0,028-0,033, bezogen auf Ascorbinsäure, α-Tocopherol als Acetat und Retinol als Acetat zusammen mit β-Carotin enthalten sind, zur Verwendung als Mittel zur Verbesserung der Verträglichkeit von Chemotherapie oder/und Strahlungstherapie maligner Tumoren.

**Claims**

1. Use of a combination of ascorbic acid, tocopherol and retinol containing ascorbic acid in a free form and tocopherol and retinol in a free form or/and in the form of pharmaceutically acceptable esters thereof in a mass ratio relative to one another of 1.5-2.5:0.3-0.8:0.028-0.033 based on ascorbic acid, α-tocopherol as an acetate and retinol as an acetate, to produce an agent for improving the tolerability of the chemotherapy or/and radiation therapy of malignant tumours.

2. Process for producing an agent which improves the tolerability of the chemotherapy or/and radiation therapy of malignant tumours,
**characterized in that**,
a combination of ascorbic acid, tocopherol and retinol in which ascorbic acid is present in a free form and tocopherol and retinol are present in a free form or/and in the form of pharmaceutically acceptable esters thereof are formulated as an active ingredient in a mass ratio relative to one another of 1.5-2.5:0.3-0.8:0.028-0.033 based on ascorbic acid, α-tocopherol as an acetate and retinol as an acetate.

3. Use as claimed in claim 1,
   **characterized in that**,
   the agent contains ascorbic acid, tocopherol and retinol in a mass ratio relative to one another of 1.8-2.1:0.5-0.65: 0.030-0.032 based on ascorbic acid, α-tocopherol as an acetate and retinol as an acetate.

4. Process as claimed in claim 2,
   **characterized in that**,
   the agent contains ascorbic acid, tocopherol and retinol in a mass ratio relative to one another of 1.8-2.1:0.5-0.65: 0.030-0.032 based on ascorbic acid, α-tocopherol as an acetate and retinol as an acetate.

5. Use as claimed in claim 1 or 2,
   **characterized in that**,
   ascorbic acid, tocopherol and retinol are administered at a minimum dose of 150 mg/day, 50 mg/day and 3 mg/day and at a maximum dose of 2.5 g/day, 800 mg/day and 33 mg/day each based on ascorbic acid, α-tocopherol as an acetate and retinol as an acetate.

6. Use as claimed in claim 5,
   **characterized in that**,
   the minimum dose of 1.5 g/day, 300 mg/day and 28 mg/day based on ascorbic acid, α-tocopherol as an acetate and retinol as an acetate is administered.

7. Use as claimed in one of the claims 1, 3, 5 and 6,
   **characterized in that**,
   the agent contains α-tocopherol or/and retinol in the form of pharmaceutically acceptable esters thereof.

8. Use as claimed in claim 7,
   **characterized in that**,
   the agent contains α-tocopherol or/and retinol in the form of acetic acid or/and succinic acid esters thereof.

9. Use as claimed in claim 7,
   **characterized in that**,
   the agent contains α-tocopherol and retinol in the form of their acetates.

10. Use as claimed in one of the claims 1, 3 and 5 to 9,
    **characterized in that**,
    the agent additionally contains pharmaceutically acceptable binding agents, flavourings, dyes, sweeteners or/and other common pharmaceutical additives.

11. Combination of ascorbic acid, tocopherol and retinol in which ascorbic acid is present in a free form and tocopherol and retinol are present in a free form or/and in the form of pharmaceutically acceptable esters thereof and they are present in a mass ratio relative to one another of 1.5-2.5:0.3-0.8:0.028-0.033 based on ascorbic acid, α-tocopherol as an acetate and retinol as an acetate for use as an agent to improve the tolerability of the chemotherapy or/and radiation therapy of malignant tumours.

12. Use of a combination of ascorbic acid, tocopherol, retinol and β-carotene in which ascorbic acid is present in a free form and tocopherol and retinol are present in a free form or/and in the form of pharmaceutically acceptable esters thereof and β-carotene is present in a free form and they are present in a mass ratio relative to one another of 1.5-2.5:0.3-0.8:0.028-0.033 based on ascorbic acid, α-tocopherol as an acetate and retinol as an acetate together with β-carotene, to produce an agent to improve the tolerability of the chemotherapy or/and radiation therapy of malignant tumours.

13. Process for producing an agent which improves the tolerability of the chemotherapy or/and radiation therapy of malignant tumours,
    **characterized in that**,
    a combination of ascorbic acid, tocopherol, retinol and β-carotene is used in which ascorbic acid in a free form and tocopherol and retinol in a free form or/and in the form of pharmaceutically acceptable esters thereof and β-carotene in a free form are formulated as an active ingredient in a mass ratio relative to one another of 1.5-2.5:0.3-0.8: 0.028-0.033 based on ascorbic acid, α-tocopherol as an acetate and retinol as an acetate together with β-carotene.

14. Use as claimed in claim 12,
**characterized in that**,
the total amount of retinol and β-carotene is composed of 1/3 retinol and 2/3 β-carotene.

15. Process as claimed in claim 13,
**characterized in that**,
the total amount of retinol and β-carotene is composed of 1/3 retinol and 2/3 β-carotene.

16. Use as claimed in claim 12,
**characterized in that**,
the dose of β-carotene is 10 mg/day to 25 mg/day.

17. Process as claimed in claim 14,
**characterized in that**,
the dose of β-carotene is 10 mg/day to 25 mg/day.

18. Combination of ascorbic acid, tocopherol, retinol and β-carotene containing ascorbic acid in a free form and tocopherol and retinol in a free form or/and in the form of pharmaceutically acceptable esters thereof and β-carotene in a free form in a mass ratio relative to one another of 1.5-2.5:0.3-0.8:0.028-0.033 based on ascorbic acid, α-tocopherol as an acetate and retinol as an acetate together with β-carotene for use as an agent to improve the tolerability of the chemotherapy or/and radiation therapy of malignant tumours.

**Revendications**

1. Utilisation d'une combinaison d'acide ascorbique, de tocophérol et de rétinol, l'acide ascorbique se présentant sous forme libre et le tocophérol et le rétinol sous forme libre et/ou sous forme de leurs esters pharmaceutiquement acceptables et étant contenus en un rapport en masse les uns aux autres de 1,5 à 2,5:0,3 à 0,8:0,028 à 0,033 par rapport à l'acide ascorbique, à l'α-tocophérol sous forme d'acétate et au rétinol sous forme d'acétate, pour produire un agent améliorant la tolérance d'une chimiothérapie et/ou d'une radiothérapie de tumeurs malignes.

2. Procédé de production d'un agent améliorant la tolérance d'une chimiothérapie et/ou d'une radiothérapie de tumeurs malignes,
**caractérisé en ce que**
l'on prépare comme principe actif une combinaison d'acide ascorbique, de tocophérol et de rétinol, l'acide ascorbique se présentant sous forme libre et le tocophérol et le rétinol sous forme libre et/ou sous forme de leurs esters pharmaceutiquement acceptables et étant contenus en un rapport en masse les uns aux autres de 1,5 à 2,5:0,3 à 0,8:0,028 à 0,033 par rapport à l'acide ascorbique, à l'α-tocophérol sous forme d'acétate et au rétinol sous forme d'acétate.

3. Utilisation selon la revendication 1,
**caractérisée en ce que**
l'agent contient l'acide ascorbique, le tocophérol et le rétinol, en un rapport en masse les uns aux autres de 1,8 à 2,1:0,5 à 0,65:0,030 à 0,032 par rapport à l'acide ascorbique, à l'α-tocophérol sous forme d'acétate et au rétinol sous forme d'acétate.

4. Procédé selon la revendication 2,
**caractérisé en ce que**
l'agent contient l'acide ascorbique, le tocophérol et le rétinol, en un rapport en masse les uns aux autres de 1,8 à 2,1:0,5 à 0,65:0,030 à 0,032 par rapport à l'acide ascorbique, à l'α-tocophérol sous forme d'acétate et au rétinol sous forme d'acétate.

5. Utilisation selon la revendication 1 ou 2,
**caractérisé en ce que**

l'acide ascorbique, le tocophérol et le rétinol sont administrés en une dose minimale de 150 mg/jour, 50 mg/jour et 3 mg/jour et en une dose maximale de 2,5 g/jour, 800 mg/jour et 33 mg/jour, respectivement, par rapport à l'acide ascorbique, à l'α-tocophérol sous forme d'acétate et au rétinol sous forme d'acétate.

**6.** Utilisation selon la revendication 5,
**caractérisée en ce que**
la dose minimale est administrée à raison de 1,5 g/jour, 300 mg/jour et 28 mg/jour par rapport à l'acide ascorbique, à l'α-tocophérol sous forme d'acétate et au rétinol sous forme d'acétate.

**7.** Utilisation selon l'une quelconque des revendications 1, 3, 5 et 6,
**caractérisée en ce que**
l'agent contient l'α-tocophérol et/ou le rétinol sous forme de leurs esters pharmaceutiquement acceptables.

**8.** Utilisation selon la revendication 7,
**caractérisée en ce que**
l'agent contient l'α-tocophérol et/ou le rétinol sous forme de leurs esters d'acide acétique et/ou d'acide succinique.

**9.** Utilisation selon la revendication 7,
**caractérisée en ce que**
l'agent contient l'α-tocophérol et le rétinol sous forme de leurs acétates.

**10.** Utilisation selon l'une quelconque des revendications 1, 3 et 5 à 9,
**caractérisée en ce que**
l'agent contient en outre des agents liants, des aromatisants, des colorants, des édulcorants et/ou additifs habituels pour les produits pharmaceutiques, pharmaceutiquement acceptables

**11.** Combinaison d'acide ascorbique, de tocophérol et de rétinol, l'acide ascorbique se présentant sous forme libre et le tocophérol et le rétinol sous forme libre et/ou sous forme de leurs esters pharmaceutiquement acceptables et étant contenus en un rapport en masse les uns aux autres de 1,5 à 2,5:0,3 à 0,8:0,028 à 0,033 par rapport à l'acide ascorbique, à l'α-tocophérol sous forme d'acétate et au rétinol sous forme d'acétate, destinée à être utilisée comme agent améliorant la tolérance d'une chimiothérapie et/ou d'une radiothérapie de tumeurs malignes.

**12.** Utilisation d'une combinaison d'acide ascorbique, de tocophérol et de rétinol et de bêta-carotène, l'acide ascorbique se présentant sous forme libre et le tocophérol et le rétinol sous forme libre et/ou sous forme de leurs esters pharmaceutiquement acceptables et le bêta-carotène sous forme libre, et étant contenus en un rapport en masse les uns aux autres de 1,5 à 2,5:0,3 à 0,8:0,028 à 0,033 par rapport à l'acide ascorbique, à l'α-tocophérol sous forme d'acétate et au rétinol sous forme d'acétate conjointement avec le bêta-carotène, pour produire un agent améliorant la tolérance d'une chimiothérapie et/ou d'une radiothérapie de tumeurs malignes.

**13.** Procédé de production d'un agent améliorant la tolérance d'une chimiothérapie et/ou d'une radiothérapie de tumeurs malignes,
**caractérisé en ce que**
l'on prépare comme principe actif une combinaison d'acide ascorbique, de tocophérol, de rétinol et de bêta-carotène, l'acide ascorbique se présentant sous forme libre et le tocophérol et le rétinol sous forme libre et/ou sous forme de leurs esters pharmaceutiquement acceptables et le bêta-carotène sous forme libre, et étant contenus en un rapport en masse les uns aux autres de 1,5 à 2,5:0,3 à 0,8:0,028 à 0,033 par rapport à l'acide ascorbique, à l'α-tocophérol sous forme d'acétate et au rétinol sous forme d'acétate, conjointement avec le bêta-carotène.

**14.** Utilisation selon la revendication 12,
**caractérisée en ce que**
la quantité totale de rétinol et de bêta-carotène est constituée d'1/3 de rétinol et de 2/3 de bêta-carotène.

**15.** Procédé selon la revendication 13,
**caractérisé en ce que**
la quantité totale de rétinol et de bêta-carotène est constituée d'1/3 de rétinol et de 2/3 de bêta-carotène.

**16.** Utilisation selon la revendication 12,
**caractérisée en ce que**

la dose de bêta-carotène est de 10 mg/jour à 25 mg/jour.

17. Procédé selon la revendication 13,
    **caractérisé en ce que**
    la dose de bêta-carotène est de 10 mg/jour à 25 mg/jour.

18. Combinaison d'acide ascorbique, de tocophérol, de rétinol et de bêta-carotène, l'acide ascorbique se présentant sous forme libre et le tocophérol et le rétinol sous forme libre et/ou sous forme de leurs esters pharmaceutiquement acceptables et le bêta-carotène sous forme libre, et étant contenus en un rapport en masse les uns aux autres de 1,5 à 2,5:0,3 à 0,8:0,028 à 0,033 par rapport à l'acide ascorbique, à l'$\alpha$-tocophérol sous forme d'acétate et au rétinol sous forme d'acétate conjointement avec le bêta-carotène, pour produire un agent améliorant la tolérance d'une chimiothérapie et/ou d'une radiothérapie de tumeurs malignes.

Figur 1

mg/l

Figur 2

Figur 3

Figur 4

Figur 5

[Monate]